# FASCICULE DE BREVET EUROPEEN

(11) **EP 1 339 468 B1**
(45) Date de publication et mention de la délivrance du brevet: **02.03.2005**
(21) Numéro de dépôt: 01998386.5
(22) Date de dépôt: 30.11.2001
(51) Int. Cl.: B01D 3/38

(54) **PROCEDE ET DISPOSITIF POUR LE TRAITEMENT EN CONTINU D'EAUX USEES D'ORIGINE INDUSTRIELLE PAR STRIPPAGE A LA VAPEUR D'EAU**
VERFAHREN UND VORRICHTUNG ZUR KONTINUIERLICHEN BEHANDLUNG VON INDUSTRIELLEM ABFALLWASSER DURCH STRIPPUNG MIT WASSERDAMPF
METHOD AND DEVICE FOR CONTINUOUSLY TREATING WASTE WATER OF INDUSTRIAL ORIGIN BY WATER VAPOUR STRIPPING

(30) Priorité: 01.12.2000 FR 0015568
(43) Date de publication de la demande: 03.09.2003
(73) Titulaire: TotalFinaElf France, 92800 Puteaux (FR)
(72) Inventeur: BUISSON, Jean-François, F-13500 Martigues (FR); POULY, Frédéric, F-69008 Lyon (FR)
(74) Mandataire: Jolly, Jean-Pierre
(86) Numéro de dépôt international: PCT/FR2001/003792
(87) Numéro de publication internationale: WO 2002/043829

(56) Documents cités:
- FR-A- 1 398 730
- US-A- 2 977 289
- US-A- 3 840 437
- US-A- 4 113 615
- US-A- 4 759 944
- US-A- 5 343 407

## Description

La présente invention concerne un procédé et un dispositif pour le traitement en continu d'eaux usées d'origine industrielle, pouvant contenir des produits contaminants, par strippage à la vapeur d'eau, c'est à dire par extraction des parties les plus volatiles contenues dans ces eaux.

On sait que, dans tous les grands pays industrialisés, les législations imposent des normes de plus en plus strictes et contraignantes concernant la qualité des eaux usées issues de différentes installations industrielles, qui sont rejetées dans le milieu naturel. En effet, ces eaux peuvent avoir été contaminées par différents polluants dangereux pour l'homme et l'environnement au cours de leurs utilisations et il est alors nécessaire et indispensable de les soumettre à un traitement approprié avant leur réintégration dans le milieu naturel.

C'est ainsi que, dans les raffineries de pétrole, qui constituent un type d'installation industrielle auquel on se référera plus précisément dans la suite de la présente description, sans que ceci ait un caractère limitatif, les eaux dites « eaux de procédé », provenant de différentes unités de la raffinerie, représentent la majorité des eaux devant subir un traitement préalable à leur réintégration dans leur milieu initial. En effet, avant de soumettre ces eaux à diverses actions physiques et biologiques de finition, on commence par leur appliquer un traitement primaire dit de « strippage » (traduction approximative de l'anglais « stripping »), qui permet d'extraire par une action physique les parties les plus volatiles qu'elles contiennent. C'est dans ce but qu'une raffinerie de pétrole est généralement équipée d'au moins un dispositif de strippage, encore appelé « stripper » dans la profession, dans lequel les eaux usées issues des différents procédés sont traitées à contre-courant par un flux de vapeur d'eau, dans une colonne renfermant plusieurs plateaux horizontaux.

Les eaux de procédé ainsi traitées, qui ne contiennent plus que des quantités limitées en plusieurs polluants tels que l'ammoniaque, les sulfures, ou différents phénols, sont évacuées à la partie inférieure du stripper, puis ensuite mélangées aux eaux de ruissellement, pour enfin être soumises à divers traitements dits de finition, avant d'être rejetées dans le milieu naturel. Parmi ces traitements, on peut mentionner:
- un pré-traitement de décantation avec écrémage de la surface, en vue d'éliminer les gouttelettes d'hydrocarbures insolubles et les plus grosses particules de matière en suspension (MES) ;
- un traitement physico-chimique de floculation et de filtration, permettant d'éliminer la plus grande partie des hydrocarbures insolubles résiduels, les petites particules de matière en suspension et les métaux dissous ou en suspension ;
- un traitement de filtration, par exemple sur lit bactérien, visant à réduire notamment la charge organique présente (carbone organique total ou COT), la teneur en phénols et la teneur en hydrocarbures dissous ;
- un traitement final de clarification.

Dans une raffinerie, le stripper disposé en amont de ces traitements complémentaires a donc une fonction primordiale, car il reçoit la quasi-totalité des eaux de procédé de diverses origines (dessalage des pétroles bruts, unités de distillation, unité de désulfuration des gazoles, craqueurs catalytiques, etc...), fortement contaminées, entre autres, par des gaz et des composés toxiques et malodorants.

Généralement, un stripper est composé d'une colonne, à l'intérieur de laquelle sont disposés des plateaux à plusieurs niveaux et dans laquelle les eaux à traiter sont introduites en tête de colonne et la vapeur d'eau en fond de celle-ci. Il existe actuellement sur le marché plusieurs variantes de stripper, notamment les dispositifs :
- sans condenseur de tête de colonne (les plus simples) ;
- avec condenseur de tête et contrôle du taux de reflux ;
- avec condenseur de tête et rebouilleur de fond, remplaçant ainsi l'injection du flux de vapeur d'eau ;
- à deux colonnes superposées, pour une meilleure efficacité du strippage des eaux fortement contaminées.

En vue de limiter et protéger les traitements complémentaires des eaux issues du stripper, et par conséquent d'éviter ou de limiter, par exemple, la détérioration du biofiltre utilisé dans l'un de ces traitements, et également pour obtenir une efficacité maximale du strippage, il est donc essentiel et même indispensable que le stripper opère dans les meilleures conditions de fonctionnement.

Afin d'optimiser un tel fonctionnement, l'exploitant peut agir sur les paramètres suivants :
- le débit d'alimentation en vapeur de strippage,
- le débit d'alimentation en eaux usées à traiter,
et, pour certaines technologies de strippers :
- le taux de reflux de l'eau de tête de colonne,
- et le taux de rebouillage en fond de colonne.

Chaque type de stripper est choisi en fonction des spécificités des sites industriels et des traitements à effectuer, conformément aux normes en vigueur sur les eaux de rejet, qui peuvent être plus ou moins chargées en contaminants.

Les réglages de ces paramètres étant directement liés à la nature et aux concentrations des contaminants présents dans les eaux de procédés, le raffineur doit donc pouvoir disposer à tout moment de telles informations pour optimiser le fonctionnement du stripper. Or il n'existe pas, à l'heure actuelle, de méthode satisfaisante pour l'analyse instantanée ou quasi-instantanée de la composition en contaminants et du degré de pollution des eaux usées arrivant au stripper. Les seules méthodes d'analyse disponibles aujourd'hui sont des méthodes de laboratoire, qui engendrent des délais de réponse importants et de nombreuses interventions humaines. En effet, les échantillons d'eaux doivent être prélevés, acheminés au laboratoire, puis analysés par différentes méthodes d'analyses, dont certaines demandent une préparation spéciale de l'échantillon. Parmi ces méthodes, on peut distinguer :
- le dosage potentiométrique, pour la mesure des sulfures,
- la distillation, puis le titrage colorimétrique, pour l'ammoniaque,
- la chromatographie en phase liquide haute performance, pour la mesure des phénols,
- l'oxydation UV, puis la détection linéaire IR, pour le Carbone Organique Total,

Sur le lieu d'exploitation, le raffineur dispose des résultats d'analyse au mieux plusieurs dizaines de minutes après la prise d'échantillons, voire plusieurs centaines de minutes, et ceci avec une fréquence limitée, par exemple moins de cinq fois par période de 24 heures de fonctionnement du stripper.

La présente invention vise donc à remédier aux inconvénients de ces différentes méthodes d'analyse de laboratoire de la technique antérieure, en proposant une analyse simultanée des différents polluants habituellement présents dans les eaux industrielles usées, par utilisation d'une seule méthode d'analyse, la spectrométrie ultraviolette, directement et automatiquement mise en oeuvre sur les eaux d'alimentation et/ou de sortie du stripper.

L'invention a également pour but de disposer en temps quasiment instantané des résultats des analyses, pour piloter en continu le stripper, par des moyens déjà décrits dans la technique, par commande et régulation de certains paramètres principaux de fonctionnement de celui-ci, tels par exemple que les débits d'alimentation en eaux usées à traiter et en vapeur d'eau de strippage.

A cet effet, la présente invention a pour objet un procédé pour le traitement en continu des eaux usées d'origine industrielle pouvant contenir divers produits contaminants, selon lequel on introduit par au moins une ligne d'alimentation les eaux usées dans une colonne de strippage dans laquelle elles s'écoulent par gravité, on injecte dans cette colonne un flux de vapeur d'eau à un niveau tel que les eaux usées et la vapeur d'eau circulent à contre-courant dans la colonne, on récupère en tête de colonne les gaz extraits par strippage des eaux usées par cette vapeur, et l'on évacue à la base de la colonne de strippage les eaux ainsi traitées, ce procédé étant caractérisé en ce que :
- on détermine en ligne au moins une portion du spectre ultra-violet des composés du groupe constitué par les sulfures, l'ammoniaque et les phénols, présents dans l'un ou l'autre des circuits d'alimentation en eaux usées ou de sortie des eaux traitées de la colonne, après dissolution de l'échantillon analysé dans une solution aqueuse tampon, pour amener son pH à une valeur supérieure à 8 et, de préférence entre 8 et 10, la spectrométrie UV étant réalisée sur la partie aqueuse de l'échantillon, après séparation de sa phase organique.
- on détermine qualitativement et quantitativement, par traitement mathématique des intensités mesurées, au moins un produit contaminant présent dans les échantillons prélevés ;
- en fonction des résultats ainsi obtenus, et par comparaison à des valeurs de consigne préalablement définies, on restitue ces informations sous forme de signaux électriques, qui commandent les débits d'alimentation en eaux usées et en vapeur d'eau de la colonne de strippage.

Les prélèvements d'échantillons des eaux, les mesures réalisées dans le domaine du spectre de la lumière ultra-violette, la transmission des signaux électriques et la commande des organes fonctionnels contrôlant les débits d'alimentation en eaux usées et en vapeur d'eau de la colonne de strippage, sont de préférence effectués par au moins un dispositif de commande automatique et programmé.

La technique d'analyse qui consiste à :
1/ utiliser un spectromètre du commerce pour la mesure du spectre UV en provenance d'un échantillon, ou une partie de ce spectre, ou une longueur d'onde précise de celui-ci, que le spectre soit émis directement ou par fluorescence, ou absorbé par cet échantillon, quand ce dernier est excité par une source lumineuse émettant dans l'Ultra-Violet,
2/ puis à appliquer aux intensités ainsi enregistrées un traitement mathématique, tel que par exemple la déconvolution de spectres ou la PLS (de l'anglais Partial Least Square),
est parfaitement connue de l'homme du métier.

On pourra, par exemple, se référer aux deux publications suivantes :
- O. Thomas, F. Theraulaz, C. Agnel et S. Suryani ("Advanced UV examination of wastewater ; Environmental Technology", 1996, vol. 17, pp 251-261) ;
- O. Thomas, F. Theraulaz, M. Domeizel et C. Massiani "UV spectral deconvolution : a valuable tool for wastewater quality determination", Environmental Technology 1993, vo1.14, pp 1187-1192).

Cette technique d'analyse connue pour son application dans un laboratoire et qui consiste à mesurer par spectrométrie UV les sulfures, l'ammoniaque ou certains phénols dans des eaux, a nécessité diverses adaptations pour son application à un processus en continu, directement sur les circuits du stripper.

C'est ainsi, par exemple, que la présence d'hydrocarbures non dissous dans les eaux de procédé entraîne une salissure très rapide de la ligne d'échantillonnage et de la cellule de mesure, perturbant ainsi la mesure par UV. C'est pour remédier à cet inconvénient qu'un coalesceur doit être installé en tête de la ligne d'alimentation en échantillon, pour ne garder, en vue de l'analyse par UV, que la phase aqueuse de l'échantillon.

De mème, le pH des eaux de procédé variant habituellement entre 4 et 10, il est connu qu'à pH 4 les sulfures qui se trouvent alors sous la forme H₂S, ne sont pas observables par spectrométrie UV. Pour garantir une analyse exhaustive de ces sulfures, l'échantillon d'eau doit donc être dilué avec une solution aqueuse tampon à pH fort, par exemple 10, de telle manière que, quelle que soit la valeur du pH de l'échantillon à analyser, sa dilution avec la solution tampon ramènera son pH au-dessus de 8, et de préférence entre 8 et 10, zone dans laquelle tous les sulfures sont observables et donc mesurables par spectrométrie UV.

Grâce au mode de pilotage en continu de la colonne de strippage utilisée dans ce procédé, il est possible de contrôler au plus près la ou les teneurs des différents contaminants contenus dans les eaux, par exemple à la sortie de la colonne, en régulant de manière automatique les débits d'alimentation en eaux usées ou en vapeur d'eau de strippage. De plus, en cas d'accroissement brutal de la teneur d'un ou plusieurs contaminants, une déviation de tout ou partie des flux d'eaux à traiter en amont du stripper peut être mise en place en temps quasiment réel, de manière automatique, vers un bac de stockage temporaire, pour l'application éventuelle d'un traitement particulier ou d'un effet de dilution.

La modification des débits d'alimentation du stripper en eaux à traiter et en vapeur, en fonction des résultats de cette analyse, s'effectue de façon conventionnelle connue de l'Homme du métier.

On notera à ce propos qu'il n'est généralement pas possible de modifier d'autres paramètres opératoires, tels que les températures et pressions des flux d'eaux entrant ou sortant du stripper. En revanche, en fonction des valeurs mesurées par le spectromètre ultra-violet disposé en ligne en amont ou en aval du stripper, l'exploitant pourra, de manière automatique ou non automatique, ajuster le pH des eaux pour une plus grande efficacité de strippage, tout en restant à l'intérieur de certaines limites admises pour la qualité des eaux de rejet dans le milieu naturel.

Le procédé conforme à l'invention est applicable à tout stripper associé à une unité de raffinerie (ou de toute autre entreprise industrielle), dans lequel sont rassemblées en amont les eaux en provenance des différentes unités et/ou des strippers secondaires qui leurs sont associés.

L'invention a également pour objet un dispositif pour la mise en oeuvre du procédé décrit ci-dessus, ce dispositif comprenant :
- une colonne de strippage des eaux ;
- au moins une ligne d'alimentation de cette colonne de strippage en eaux à traiter, cette alimentation s'effectuant à un niveau tel que les eaux s'écoulent par gravité vers le fond de la colonne ;
- au moins une ligne d'alimentation de la colonne de strippage en vapeur d'eau destinée à stripper les gaz contenus dans les eaux à traiter, l'alimentation en vapeur d'eau s'effectuant à un niveau tel que les eaux usées et la vapeur d'eau circulent à contre-courant dans la colonne ;
- à la partie supérieure de la colonne, au moins une ligne d'évacuation de la vapeur d'eau et des gaz extraits par strippage des eaux usées par cette vapeur, avec éventuellement un moyen de séparation des gaz et de la vapeur ;
- à la partie inférieure de la colonne, au moins une ligne d'évacuation des eaux traitées ;
- sur les lignes d'alimentation de la colonne de strippage en eaux usées et en vapeur d'eau, des organes commandés de réglage du débit ; ce dispositif étant caractérisé en ce qu'il comprend :
   - sur au moins une ligne d'alimentation en eaux usées ou sur au moins la ligne d'évacuation des eaux traitées, un moyen commandé de prélèvement d'un échantillon des eaux circulant dans cette ligne ;
   - connecté à ces moyens de prélèvement d'échantillon, un moyen d'analyse par spectrométrie ultra-violette et un moyen de calcul associé, pour déterminer la présence et la quantité des contaminants éventuellement présents dans les échantillons prélevés ;
   - connecté à ce moyen de calcul, un dispositif programmé d'actionnement des organes fonctionnels de commande du débit des lignes d'alimentation de la colonne de strippage en eaux usées et en vapeur d'eau, en fonction des résultats d'analyse des échantillons.

Comme indiqué ci-dessus, ce dispositif peut avantageusement comporter également des moyens de mesure d'autres paramètres opératoires, tels que la température et la pression des flux d'eaux entrant et sortant de la colonne de strippage, et des moyens de transmission de ces mesure au dispositif de commande des organes fonctionnels de la colonne de strippage.

D'autres caractéristiques et avantages de l'invention, dans son application au traitement des eaux usées d'une partie d'une raffinerie de pétrole comprenant une colonne de distillation atmosphérique et diverses installations annexes, apparaîtront dans la description détaillée qui va suivre. Dans cette description, on se référera aux dessins annexés, dans lesquels :
La figure 1 est une vue schématique partielle des circuits d'eaux usées de la raffinerie, illustrant la position de la colonne principale de strippage à la vapeur d'eau des gaz contenus dans ces eaux usées ;
La figure 2 est une vue schématique partielle, illustrant le système de pilotage conforme à l'invention de la colonne de strippage.

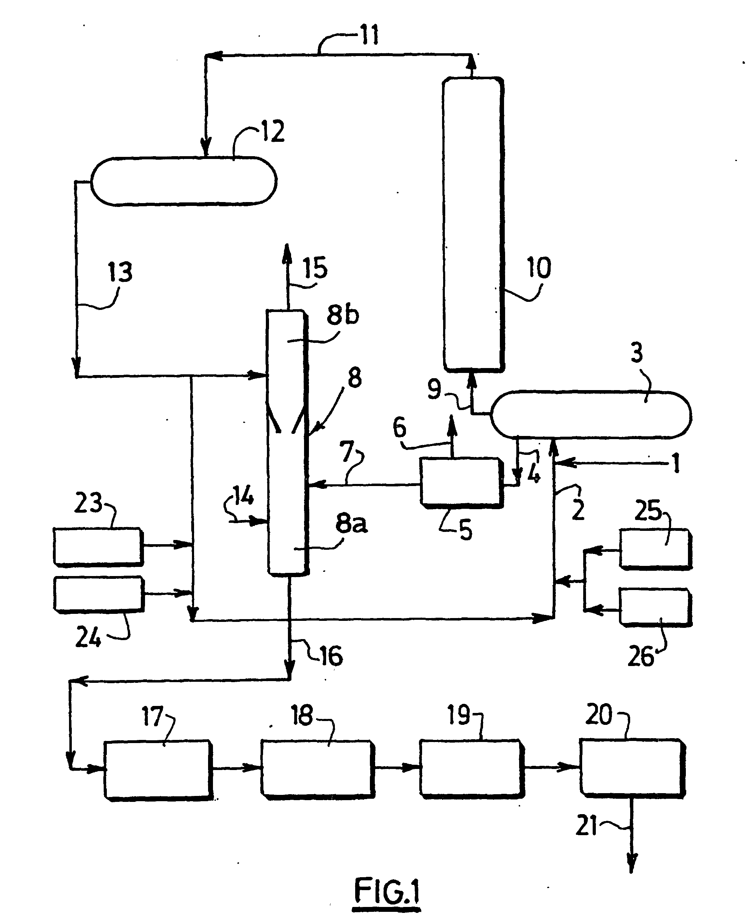

On se référera d'abord à la figure 1, sur laquelle, pour simplifier, seuls certains des strippers primaires, associés de préférence à chacune des unités, ont été schématisés.

L'alimentation en pétrole brut et en eau de dessalement du dessaleur 3 se fait respectivement par les lignes 1 et 2. L'eau salée issue du dessaleur 3 passe par la ligne 4 dans un décanteur 5, d'où les hydrocarbures liquides présents sont évacués par la ligne 6, tandis que l'eau salée récupérée par la ligne 7 est injectée à 1a partie inférieure 8a d'une colonne de strippage 8 qui, dans cet exemple, est à deux étages 8a et 8b.

Le pétrole brut issu du dessaleur 3 est introduit par la ligne 9 dans une colonne de distillation atmosphérique 10, dont les différentes lignes d'évacuation des coupes séparées et les installations annexes n'ont pas été représentées.

Les vapeurs récupérées en tête de colonne sont dirigées par la ligne 11 vers un ballon de condensation 12, d'où les eaux condensées sont acheminées par la ligne 13 vers la partie supérieure 8b de la colonne de strippage 8.

La ligne 2 d'alimentation en eau du dessaleur est raccordée à la ligne 13 et divers appoints d'eau y sont introduits, par exemple en provenance d'un bac de stockage 23 d'eaux de diverses origines, d'un bac de condensation 24 des effluents de tête d'une colonne de séchage de gazoles, et de divers strippers primaires associés à des unités de l'installation, par exemple un stripper 25 d'une unité de désulfuration de gazoles et un stripper 26 d'une unité de craquage catalytique.

Dans la colonne 8, les eaux introduites en 7 et en 13 s'écoulent par gravité vers le fond, à contre-courant d'un flux de vapeur d'eau introduit à un niveau inférieur par la ligne 14, et les gaz dissous dans. ces eaux sont strippés par la vapeur d'eau et évacués en 15 avec la vapeur restante à la partie supérieure de la colonne 8. La vapeur d'eau peut être générée par un dispositif de rebouillage en continu disposé au fond de colonne 8.

Les eaux traitées sont évacuées par la ligne 16 à la partie inférieure de la colonne 8 et passent successivement dans un décanteur 17, sur des filtres à sable 18, dans un biofiltre 19 et à nouveau dans un bassin de clarification 20, avant d'être évacuées dans le milieu naturel par la ligne 21.

Le biofiltre 19 est un biofiltre bactérien aérobie, à ruissellement et à garnissage ordonné, qui est destiné à assurer la destruction par des micro-organismes de la matière organique dissoute, pour la transformer en biomasse, en gaz carbonique et en eau. Ce biofiltre assure également l'élimination quasi-complète du sulfure d'hydrogène présent, principalement par aération.

Les bactéries de ce biofiltre sont très sensibles à certains polluants, qui sont toxiques à leur égard au-delà d'une certaine teneur, égale par exemple à 8 mg/1 pour les sulfures. Il importe donc d'éliminer ces produits contaminants ou d'abaisser leur concentration au-dessous des seuils limites en amont du biofiltre et, en particulier, au niveau du stripper.

Comme indiqué ci-dessus, l'un des buts de la présente invention est précisément de piloter en continu le fonctionnement de ce stripper, en mesurant les teneurs en divers polluants des eaux usées à traiter et/ou celles des eaux traitées et en faisant varier en conséquence les débits d'alimentation du stripper en ces eaux, en vapeur d'eau et éventuellement en vapeur d'eau condensée recyclée, de manière à opérer dans les conditions de purification des eaux les plus efficaces.

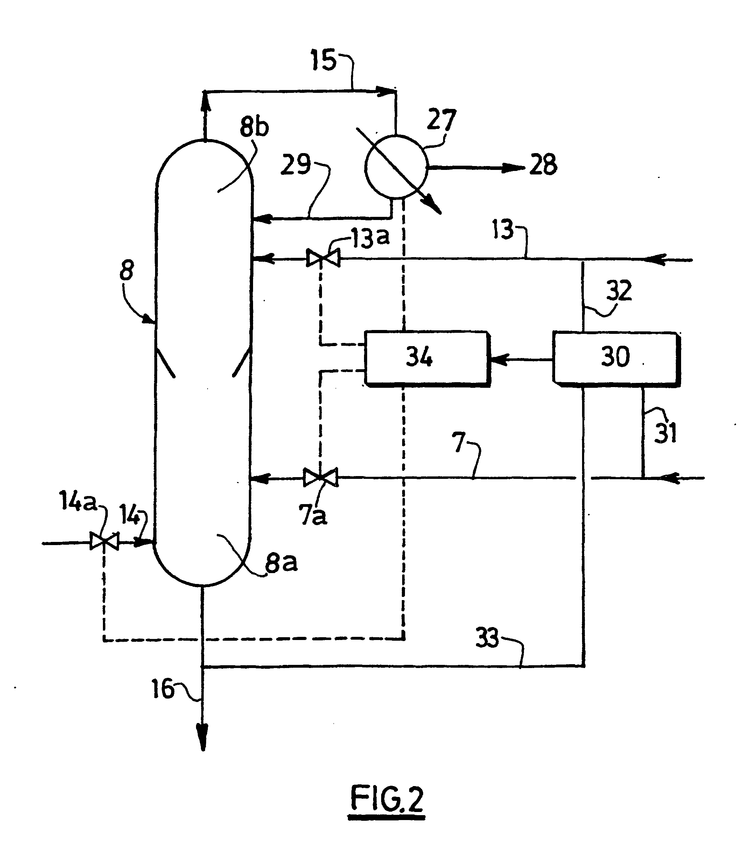

C'est ce qui va maintenant être exposé en référence à la figure 2.

On retrouve sur cette figure 2 la colonne de strippage 8 de la figure 1, la ligne 7 d'introduction dans cette colonne des eaux issues du dessalement du pétrole brut, la ligne 13 d'introduction d'autres eaux usées à traiter, la ligne 14 d'injection de la vapeur d'eau, la ligne 15 d'évacuation en tête de colonne de la vapeur d'eau et des gaz strippés par cette vapeur, et la ligne 16 d'évacuation des eaux traitées en fond de colonne.

Dans cette configuration, la ligne 15 alimente un ballon de condensation 27, d'où les gaz strippés sont évacués par la ligne 28, tandis que l'eau provenant de la condensation de la vapeur est recyclée par la ligne 29 à un taux de reflux réglable en tête de colonne.

Les lignes 7, 13 et 14, comportent des vannes, respectivement 7a, 13a et 14a, permettant de régler le débit d'alimentation de la colonne 8 en les fluides qui y circulent.

Conformément à l'invention, un analyseur 30 par spectrométrie ultra-violette est connecté aux lignes 7, 13 et 16, respectivement, par des lignes 31, 32 et 33, afin d'effectuer des prélèvements commandés d'échantillons des fluides circulant dans ces lignes et de déterminer la teneur en certains contaminants présents dans ces échantillons. Les analyseurs de ce type sont bien connus dans la technique et sont commercialisés par exemple sous l'appellation IXO 510 par la société SECOMAN.

Les spectres obtenus sont traités par déconvolution spectrale dans une centrale de commande 34 programmée, c'est à dire qu'ils sont décomposés en un certain nombre de spectres des produits contaminants sur lesquels on recherche des informations (sulfures, chlorures, carbone organique total ou COT, matières en suspension ou MES, ammoniaque, etc). Les spectres obtenus sont automatiquement comparés à des spectres d'une base de référence constituée d'échantillons connus, en vue de déterminer la teneur des échantillons en ces contaminants. Cette méthode d'analyse par déconvolution est décrite par exemple par S. Gallot et O. Thomas dans « State of the art for the examination of UV spectra of waters and wastewaters », Intern. J. Environ. Anal. Chem., vol. 52, pp 149-158.

La centrale de commande 34 comprend à cet effet un programme de commande qui, en fonction des résultats d'analyse, agit sur les vannes 7a, 13a, 14a et sur le condenseur 27, pour modifier de façon appropriée les débits d'alimentation de la colonne 8 en eaux usées et en vapeur d'eau, ainsi que le taux de reflux d'eau en tête de la colonne.

Les prélèvements d'échantillons peuvent être commandés par un opérateur ou automatiquement, à des intervalles réguliers, par la centrale de commande, par exemple toutes les dix minutes.

On notera la grande simplicité de mise en oeuvre du procédé conforme à l'invention. La durée des analyses est en général d'environ 5 minutes et le pilotage du stripper peut, par conséquent, s'effectuer immédiatement après ces analyses.

Bien que l'invention ait été décrite essentiellement dans son application au traitement des eaux usées d'une raffinerie, il sera clair, pour l'homme du métier, qu'elle s'applique également au traitement de tout autre type d'eaux usées, en provenance d'installations industrielles différentes.

## Revendications

1. Procédé pour le traitement en continu d'eaux usées d'origine industrielle pouvant contenir divers produits contaminants, selon lequel on introduit par au moins une ligne d'alimentation (7, 13) les eaux usées dans une colonne de strippage (8) dans laquelle elles s'écoulent par gravité, on injecte (en 14) dans cette colonne (8) un flux de vapeur d'eau à un niveau tel que les eaux usées et la vapeur d'eau circulent à contre-courant dans la colonne (8), on récupère en tête de colonne (en 15) les gaz extraits par strippage des eaux usées par cette vapeur, et l'on évacue (en 16) à la base de la colonne (8) de strippage les eaux ainsi traitées, ce procédé étant **caractérisé en ce que** :
- on détermine en ligne au moins une portion du spectre ultra-violet des composés du groupe constitué par les sulfures, l'ammoniaque et les phénols, présents dans l'un ou l'autre des circuits d'alimentation (7, 13) en eaux usées ou de sortie des eaux traitées (16) de la colonne (8), après dissolution de l'échantillon analysé dans une solution aqueuse tampon, pour amener son pH à une valeur supérieure à 8, et de préférence, entre 8 et 10, la spectrométrie UV étant réalisée sur la partie aqueuse de l'échantillon, après séparation de sa phase organique ;
- on détermine qualitativement et quantitativement, par traitement mathématique des intensités mesurées, au moins un produit contaminant présent dans les échantillons prélevés ;
- en fonction des résultats ainsi obtenus et par comparaison à des valeurs de consigne préalablement définies, on restitue ces informations sous forme de signaux électriques, qui commandent les débits d'alimentation en eaux usées et en vapeur d'eau de la colonne de strippage.

2. Procédé selon la revendication 1, **caractérisé en ce que** ces prélèvements d'échantillons d'eaux, leur analyse par spectrométrie ultra-violette et la transmission des signaux de commande pour le réglage et la régulation des débits d'alimentation de la colonne (8) de strippage en eaux à traiter et en vapeur d'eau, sont commandés par au moins un dispositif automatique programmé (30, 34).

3. Procédé selon l'une des revendications 1 et 2, **caractérisé en ce que** le pH des eaux à traiter est ajusté en fonction des résultats des mesures par spectrométrie ultra-violette.

4. Procédé selon l'une des revendications 1 à 3, **caractérisé en ce que** les eaux usées à traiter sont introduites dans la colonne de strippage à au moins deux niveaux différents (7, 13) de celle-ci.

5. Procédé selon l'une des revendications 1 à 4, **caractérisé en ce que** les gaz extraits des eaux usées par strippage sont séparés de la vapeur d'eau dans un ballon de condensation (27) et évacués (en 28) pour un éventuel traitement ultérieur.

6. Procédé selon la revendication 5, **caractérisé en ce que** l'eau provenant de la condensation de la vapeur dans le ballon de condensation est recyclée (29) en tête de la colonne (8) avec un taux de reflux réglable

7. Procédé selon l'une des revendications 1 à 6, **caractérisé en ce que** la vapeur introduite en bas de colonne (8a) est générée par un dispositif de rebouillage en continu disposé en fond de colonne (8).

8. Procédé selon la revendication 1 à 7, **caractérisé en ce que** les eaux usées à traiter sont introduites dans la partie inférieure (8a) de la colonne de strippage et dans la partie supérieure (8b) de celle-ci.

9. Dispositif pour la mise en oeuvre du procédé selon l'une des revendications précédentes, ce dispositif comprenant :
- une colonne de strippage (8) des eaux à traiter,
- au moins une ligne d'alimentation (7, 13) de cette colonne de strippage (8) en eaux à traiter, cette alimentation s'effectuant à un niveau tel que les eaux s'écoulent par gravité vers le fond de la colonne ;
- au moins une ligne d'alimentation (14) de la colonne de strippage (8) en vapeur d'eau destinée à stripper les gaz contenus dans les eaux à traiter, l'alimentation en vapeur d'eau s'effectuant à un niveau tel que les eaux usées et la vapeur d'eau circulent à contre-courant dans la colonne (8) ;
- à la partie supérieure de la colonne (8), au moins une ligne d'évacuation (15) de la vapeur d'eau et des gaz extraits par strippage des eaux usées par cette vapeur, avec éventuellement un moyen de séparation des gaz et de la vapeur d'eau (27) ;
- à la partie inférieure de la colonne (8), au moins une ligne d'évacuation (16) des eaux traitées ;
- sur les lignes d'alimentation (7, 13, 14) de la colonne de strippage en eaux usées et en vapeur d'eau, des organes commandés de réglage du débit (7a, 13a, 14a) ; ce dispositif étant **caractérisé en ce qu'**il comprend :
- sur au moins une ligne d'alimentation en eaux usées (7, 13) ou sur au moins la ligne d'évacuation des eaux traitées (16), un moyen commandé de prélèvement d'un échantillon des eaux circulant dans cette ligne ;
- connecté à ce moyen de prélèvement d'échantillon, un moyen (30) d'analyse par spectrométrie ultra-violette et un moyen de calcul associé, pour déterminer la présence et la quantité des contaminants du groupe constitué par les sulfures, l'ammoniaque et les phénols éventuellement présents dans les échantillons prélevés ;
- connecté à ce moyen de calcul (30), un dispositif programmé d'actionnement des organes fonctionnels de commande du débit des lignes d'alimentation de la colonne de strippage en eaux usées et en vapeur d'eau, en fonction des résultats d'analyse des échantillons.

10. Dispositif selon la revendication 9, **caractérisé en ce qu'**il comprend un moyen de commande programmé des moyens de prélèvement des échantillons des eaux usées et des eaux traitées.

11. Dispositif selon l'une des revendications 9 et 10, **caractérisé en ce que** la colonne de strippage (8) comporte deux étages distincts (8a, 8b) recevant chacun une partie des eaux à traiter.

12. Dispositif selon l'une des revendications 9 à 11, **caractérisé en ce qu'**un ballon de condensation (21) est disposé en tête de colonne (8), en vue de séparer les gaz strippés et la vapeur d'eau.

13. Dispositif selon la revendication 12, **caractérisé en ce que** le taux de reflux en tête de la colonne (8) de l'eau de condensation de la vapeur d'eau est réglable.

14. Dispositif selon les revendications 9 à 13, **caractérisé en ce qu'**un rebouilleur est disposé en fond de colonne (8), en vue de transformer l'eau de condensation en vapeur d'eau

15. Procédé selon l'une des revendications 1 à 8, **caractérisé en ce que** les eaux usées proviennent d'une raffinerie de pétrole.

## Patentansprüche

1. Verfahren zur kontinuierlichen Behandlung von Industrieabwasser, das verschiedene Verschmutzungen enthalten kann, wobei
das Abwasser über zumindest eine Zuführleitung (7, 13) in eine Strippsäule (8) eingeleitet wird, in der sie durch die Schwerkraft abfließen,
in diese Säule (8) ein Wasserdampfstrom auf so einem Niveau eingespritzt wird (bei 14), dass das Abwasser und der Wasserdampf in der Säule (8) im Gegenstrom zirkulieren,
an der Spitze der Säule (bei 15) die durch Strippen des Abwassers durch diesen Dampf herausgezogenen Gase rückgewonnen werden, und
das so behandelten Wasser an dem Boden der Strippsäule (8) abgelassen wird (bei 16),
wobei das Verfahren **dadurch gekennzeichnet ist, dass**
als Linie zumindest ein Abschnitt des ultravioletten Spektrums von Verbindungen aus der durch Sulfide, Ammoniak und Phenole gebildeten Gruppe bestimmt wird, die in dem einen oder in dem anderen der Zuführkreise (7, 13) des Abwassers oder dem Ablass des behandelten Wassers (16) der Säule (8) vorhanden sind, nach dem Lösen der analysierten Probe in einer wässrigen Pufferlösung, um ihren pH-Wert auf einen höheren Wert als 8 und vorzugsweise zwischen 8 und 10 zu bringen, wobei die UV-Spektrometrie an dem wässrigen Teil der Probe nach der Abtrennung der organischen Phase durchgeführt wird,
qualitativ und quantitativ durch mathematische Verarbeitung der gemessenen Intensitäten zumindest ein Verunreinigungsprodukt bestimmt wird, das in den entnommenen Proben enthalten ist,
diese Informationen in Abhängigkeit von den so gewonnenen Ergebnissen und durch Vergleich mit vorbestimmten Einstellwerten in Form von elektrischen Signalen abgebildet werden, die die Zuführdurchsätze der Strippsäule an Abwasser und Wasserdampf steuern.

2. Verfahren nach Anspruch 1, **dadurch gekennzeichnet, dass** die Entnahme der Wasserproben, ihre Analyse durch Ultraviolettspektrometrie und die Übertragung der Steuersignale für die Einstellung und die Regulierung der Zuführdurchsätze der Strippsäule (8) an zu behandelndem Wasser und Wasserdampf durch zumindest eine automatische programmierte Vorrichtung (30, 34) gesteuert werden.

3. Verfahren nach Anspruch 1 oder 2, **dadurch gekennzeichnet, dass** der pH-Wert des zu behandelnden Wassers in Abhängigkeit von den Ergebnissen der Messungen durch die Ultraviolettspektrometrie eingestellt wird.

4. Verfahren nach einem der Ansprüche 1 bis 3, **dadurch gekennzeichnet, dass** das zu behandelnde Abwasser auf zumindest zwei unterschiedlichen Niveaus (7, 13) der Strippsäule (8) in diese eingeleitet wird.

5. Verfahren nach einem der Ansprüche 1 bis 4, **dadurch gekennzeichnet, dass** die aus dem Abwasser durch Strippen herausgezogenen Gase in einem Kondensationskolben (27) von dem Wasserdampf getrennt werden und für eine eventuelle nachfolgende Behandlung abgelassen werden (bei 28).

6. Verfahren nach Anspruch 5, **dadurch gekennzeichnet, dass** das aus der Kondensation des Dampfes in dem Kondensationskolben stammende Wasser an der Spitze der Säule (8) mit einem einstellbaren Rückflussanteil wiederzugeführt wird (bei 29).

7. Verfahren nach einem der Ansprüche 1 bis 6, **dadurch gekennzeichnet, dass** der unten in die Säule (8a) eingeführte Dampf durch eine kontinuierliche Wiederaufkochvorrichtung erzeugt wird, die am Boden der Säule angeordnet ist.

8. Verfahren nach einem der Ansprüche 1 bis 7, **dadurch gekennzeichnet, dass** das zu behandelnde Abwasser in den unteren Abschnitt (8a) der Strippsäule und in ihren oberen Abschnitt (8b) eingeleitet wird.

9. Vorrichtung zum Durchführen des Verfahrens nach einem der vorigen Ansprüche, wobei die Vorrichtung enthält:
eine Strippsäule (8) für das zu behandelnde Wasser,
zumindest eine Zuführleitung (7, 13) der Strippsäule (8) für zu behandelndes Wasser, wobei die Zufuhr auf so einem Niveau durchgeführt wird, das das Wasser durch die Schwerkraft zu dem Boden der Säule hin abfließt,
zumindest eine Zuführleitung (14) der Strippsäule (8) für Wasserdampf, der zum Strippen der in dem zu behandelnden Wasser enthaltenen Gase bestimmt ist, wobei die Zufuhr des Wasserdampfs auf so einem Niveau durchgeführt wird, dass das Abwasser und der Wasserdampf in der Säule (8) im Gegenstrom zirkulieren,
zumindest eine Ablassleitung (15) für den Wasserdampf und die durch Strippen des Abwassers durch diesen Dampf herausgezogenen Gase an dem oberen Abschnitt der Säule (8), eventuell mit einem Mittel (27) zum Trennen der Gase und des Wasserdampfs,
zumindest eine Ablassleitung (16) für das behandelte Wasser an dem unteren Abschnitt der Säule (8),
gesteuerte Durchflusseinstellorgane (7a, 13a, 14a) an den Zuführleitungen (7, 13, 14) der Strippsäule für Abwasser und Wasserdampf;
wobei die Vorrichtung **dadurch gekennzeichnet ist, dass** sie enthält:
zumindest an einer Zuführleitung (7, 13) für das Abwasser oder an der Ablassleitung (16) für das behandelte Wasser ein gesteuertes Mittel zur Entnahme einer Probe des in dieser Leitung zirkulierenden Wassers,
verbunden mit diesem Mittel zur Entnahme einer Probe ein Mittel (30) zur Analyse durch Ultraviolettspektrometrie und ein zugeordnetes Berechnungsmittel, um das Vorhandensein und die Menge von Verunreinigungen einer aus Sulfiden, Ammoniak und Phenolen gebildeten Gruppe zu bestimmen, die eventuell in den entnommenen Proben vorhanden sind,
verbunden mit dem Berechnungsmittel (30) eine programmierte Vorrichtung zur Betätigung von Funktionsorganen zum Steuern des Durchflusses der Zuführleitungen der Strippsäule für Abwasser und Wasserdampf in Abhängigkeit von den Analyseergebnissen der Proben.

10. Vorrichtung nach Anspruch 9, **dadurch gekennzeichnet, dass** sie ein programmiertes Steuermittel für die Mittel zur Entnahme von Proben von Abwasser und behandeltem Wasser enthält.

11. Vorrichtung nach Anspruch 9 oder 10, **dadurch gekennzeichnet, dass** die Strippsäule (8) zwei verschiedene Etagen (8a, 8b) aufweist, von denen jede einen Teil des zu behandelnden Wassers empfängt.

12. Vorrichtung nach einem der Ansprüche 9 bis 11, **dadurch gekennzeichnet, dass** ein Kondensationskolben (21) an der Spitze der Säule (8) bereitgestellt ist, um die gestrippten Gase und den Wasserdampf zu trennen.

13. Vorrichtung nach Anspruch 12, **dadurch gekennzeichnet, dass** der Rückflussanteil des Kondensationswassers aus dem Wasserdampf an der Spitze der Säule (8) einstellbar ist.

14. Vorrichtung nach einem der Ansprüche 9 bis 13, **dadurch gekennzeichnet, dass** ein Wiederaufkocher am Boden der Säule (8) angeordnet ist, um das Kondensationswasser in Wasserdampf umzuwandeln.

15. Verfahren nach einem der Ansprüche 1 bis 8, **dadurch gekennzeichnet, dass** das Abwasser aus einer Ölraffinerie stammt.

## Claims

1. A process for the continuous treatment of waste water of industrial origin, which may contain various contaminating products, according to which the waste water is introduced through at least one supply line (7,13) into a stripping column (8) in which it flows under gravity, a flow of steam is injected (at 14) into said column (8) at a level such that the waste water and the steam circulate in counterflow in the column (8), at the top of the column (at 15) the gases extracted by stripping the waste water with said steam are recovered and the water thus treated is discharged (at 16) at the base of the stripping column (8), said process being **characterised by**:
- determining in line at least one portion of the ultraviolet spectrum of the compounds of the group comprising the sulphides, ammonia and the phenols, which are present in one or other of the circuits for supplying waste water (7,13) or for the outlet of treated water (16) from the column (8), after dissolution of the sample analysed in an aqueous buffer solution, so as to bring its pH value above 8 and, preferably, between 8 and 10, the UV spectrometry being carried out on the aqueous part of the sample, after separation of its organic phase;
- determining qualitatively and quantitatively, by mathematical processing of the intensities measured, at least one contaminating product present in the samples taken;
- as a function of the results thus obtained and by comparison with previously defined rated values, reproducing this data in the form of electrical signals which control the rates of flow of waste water and steam supplying the stripping column.

2. A process according to claim 1, **characterised in that** this taking of water samples, their analysis by ultraviolet spectrometry and the transmission of control signals for adjusting and regulating the flow rates of water to be treated and steam supplying the stripping column (8) are controlled by at least one programmed automatic device (30,34).

3. A process according to either one of claims 1 and 2, **characterised in that** the pH value of the water to be treated is adjusted as a function of the results of the ultraviolet spectrometry measurements.

4. A process according to any one of claims 1 to 3, **characterised in that** the waste water to be treated is introduced into the stripping column at at least two different levels (7,13) thereof.

5. A process according to any one of claims 1 to 4, **characterised in that** the gases extracted from the waste water by stripping are separated from the steam in a condensation flask (27) and are discharged (at 28) for possible subsequent treatment.

6. A process according to claim 5, **characterised in that** the water arising from the condensation of the steam in the condensation flask is recycled (29) at the top of the column (8) at an adjustable reflux rate.

7. A process according to any one of claims 1 to 6, **characterised in that** the steam introduced at the base of the column (8a) is generated by a continuous reboiling device arranged at the bottom of the column (8).

8. A process according to any one of claims 1 to 7, **characterised in that** the waste water to be treated is introduced into the lower part (8a) of the stripping column and into the upper part (8b) thereof.

9. An apparatus for carrying out the process according to any one of the preceding claims, said apparatus comprising:
- a stripping column (8) for the water to be treated,
- at least one line (7,13) for supplying said stripping column (8) with water to be treated, this supply taking place at a level such that the water flows under gravity towards the bottom of the column;
- at least one line (14) for supplying the stripping column (8) with steam intended to strip the gases contained in the water to be treated, the supply of steam taking place at a level such that the waste water and the steam circulate in counterflow in the column (8);
- in the upper part of the column (8) at least one discharge line (15) for steam and the gases extracted by stripping the waste water with said steam, optionally with a means (27) for separating the gases and the steam;
- in the lower part of the column (8) at least one discharge line (16) for treated water;
- in the supply lines (7,13,14) of the stripping column with waste water and steam, control members (7a,13, 14a) for regulating the flow rate; said apparatus being **characterised in that** it comprises:
- in at least one waste-water supply line (7,13) or in at least one discharge line (16) for treated water, a controlled means for taking a sample of the water circulating in said line;
- connected to said sampling means, a means (30) for analysing by ultraviolet spectrometry and an associated calculating means, for determining the presence and quantity of contaminants of the group comprising the sulphides, ammonia and the phenols, which may be present in the samples taken;
- connected to said calculating means (30), a programmed actuating device for the operating members for controlling the flow rate of waste water and steam in the lines supplying the stripping column, as a function of the results of the sample analysis.

10. An apparatus according to claim 9, **characterised in that** it comprises a programmed control means for the means for taking samples from the waste water and treated water.

11. An apparatus according to either one claims 9 and 10, **characterised in that** the stripping column (8) comprises two separate stages (8a,8b) each receiving part of the water to be treated.

12. An apparatus according to any one of claims 9 to 11, **characterised in that** a condensation flask (21) is arranged at the top of the column (8) and is for separating the stripped gases and the steam.

13. An apparatus according to claim 12, **characterised in that** the reflux rate at the top of the column (8) of the water of condensation from the steam is adjustable.

14. An apparatus according to any one of claims 9 to 13, **characterised in that** a reboiler is arranged at the bottom of the column (8), and is for converting the water of condensation into steam.

15. A process according to any one of claims 1 to 8, **characterised in that** the waste water originates from a petroleum refinery.
